# EUROPEAN PATENT APPLICATION

(11) **EP 3 424 415 A1**
(43) Date of publication of application: **09.01.2019**
(21) Application number: 17759640.0
(22) Date of filing: 14.02.2017
(51) Int. Cl.: A61B 5/02, A61B 5/00, A61B 5/0245

(54) **BIOLOGICAL INFORMATION EXTRACTING DEVICE AND BIOLOGICAL INFORMATION EXTRACTING SYSTEM**

(30) Priority: 03.03.2016 JP 2016040890
(71) Applicant: Panasonic Intellectual Property Management Co., Ltd., Osaka-shi, Osaka 540-6207 (JP)
(72) Inventor: NAKAMURA Tsuyoshi, Osaka 540-6207 (JP); MATSUO Masatoshi, Osaka 540-6207 (JP); TEZUKA Tadanori, Osaka 540-6207 (JP); OHASHI Masahiro, Osaka 540-6207 (JP)
(74) Representative: Eisenführ Speiser
(86) International application number: PCT/JP2017/005214
(87) International publication number: WO 2017/150169

(57) **Abstract**

A biological information extracting device which is provided at a head-mounted member mounted on a head of a person (subject) and includes a camera that captures temporally successive images including at least a part of a face of the person as an object; a region selector that selects a skin color region from the image captured by the camera; a pulse wave measurer that measures a pulse wave signal of the person based on image data of the skin color region; and a data transmitter that transmits the pulse wave signal to a predetermined transmission destination. The camera is disposed to face a side surface of the face of the person and is set to capture a part (cheek of the face) of the side surface of the face.

## Description

### TECHNICAL FIELD

The present disclosure relates to a biological information extracting device and a biological information extracting system for extracting biological information from information obtained without coming into contact with a human body.

### BACKGROUND ART

Regarding measurement of a pulse of a person, there are a method in which a measuring person (nurse or the like) puts his/her finger on a wrist of a subject to personally check pulsation, a method in which a dedicated measuring instrument is attached to a wrist, a finger, or the like of a subject to automatically measure pulsation, and the like. On the other hand, in such a measuring method, since free movement of the subject is temporarily restricted or it is necessary to attach the measuring instrument to the subject, a technique for estimating (measuring) a pulse without coming into contact with the subject (human body) has been developed. For example, a technique for extracting a pulse wave signal that is time series data of a pulse wave of the subject from image data obtained by capturing the subject and estimating (measuring) the pulse based on the pulse wave signal is known (see PTL 1).

In addition, for example, at a construction site, a building site, or the like, in order to prevent occurrence of accident caused by a health condition (for example, drowsiness, stress, or poor condition) of a worker, it is preferable that the biological information (for example, the pulse wave or the pulse) of the worker is obtained and the health condition of the worker is monitored and managed based on the biological information. Therefore, a technique for determining a health condition by capturing a front surface of a face of the subject by a camera installed in a flange of a front surface of a helmet mounted on a head of the subject (measurement target person), wirelessly transmitting the image data (video information) to a management computer, and extracting the biological information of the subject based on the image data in the management computer is proposed (see PTL 2).

However, when the image data obtained by capturing the front surface of the face of the subject is created and transmitted, in a case where a trouble such as erroneous transmission or information leakage occurs, a privacy of the subject cannot be protected. In addition, in a case where the subject wears a shielding object such as sunglasses or a mask on the face, the biological information cannot be accurately extracted based on the image obtained by capturing the front surface of the face of the subject, and in some cases, the biological information cannot be extracted.

Furthermore, since the image data has a large data amount, in order to secure a large amount of a communication band or to reduce a transmission data amount, it is necessary to compress the image data or reduce an image size by using a known image compression technique such as H. 264. However, in a case where the image data is compressed or the image size is reduced, extraction accuracy of the biological information based on the image data is lowered.

An object of the present disclosure is to protect a privacy of a subject, to extract biological information of the subject even in a case where the subject wears a shielding object such as sunglasses or a mask on a face, and to reduce a transmission data amount without lowering extraction accuracy of the biological information.

### Citation List

### Patent Literature

PTL 1: Japanese Patent Unexamined Publication No. 2012-239661
PTL 2: International Publication No. 2015/098977

### SUMMARY OF THE INVENTION

A biological information extracting device of the present disclosure which is provided at a head-mounted member mounted on a head of a subject and extracts biological information of the subject from information obtained without coming into contact with the subject including: a camera that captures temporally successive images including at least a part of a face of the subject as an object; a region selector that selects a skin color region from the image captured by the camera; and a data transmitter that transmits biomaterial-related data relating to a biomaterial of the subject extracted based on the skin color region to a predetermined transmission destination. The camera is disposed to face a side surface of the face of the subject.

According to the present disclosure, it is possible to protect a privacy of a subject, to extract biological information of the subject even in a case where the subject wears a shielding object such as sunglasses or a mask on the face, and to reduce a transmission data amount without lowering extraction accuracy of the biological information.

### BRIEF DESCRIPTION OF DRAWINGS

FIG. 1 is an entire configuration view of a biological information extracting system according to a first embodiment.
FIG. 2 is a function block diagram of the biological information extracting system according to the first embodiment.
FIG. 3A is an explanatory view of a pulse wave measuring process and a pulse estimating process.
FIG. 3B is an explanatory view of a pulse wave measuring process and a pulse estimating process.
FIG. 4A is a block diagram illustrating a hardware configuration for realizing a biological information extracting device according to the first embodiment.
FIG. 4B is a block diagram illustrating a hardware configuration for realizing a biological information extracting device according to the first embodiment.
FIG. 5 is a flowchart illustrating a flow of a process by the biological information extracting device according to the first embodiment.
FIG. 6 is a flowchart illustrating a flow of a process by an information analyzing device according to the first embodiment.
FIG. 7A is a view illustrating a result of an experiment in which a relationship between a shielding object worn on a face of a person and a heart rate of the person is examined.
FIG. 7B is a view illustrating a result of an experiment in which a relationship between a shielding object worn on a face of a person and a heart rate of the person is examined.
FIG. 7C is a view illustrating a result of an experiment in which a relationship between a shielding object worn on a face of a person and a heart rate of the person is examined.
FIG. 7D is a view illustrating a result of an experiment in which a relationship between a shielding object worn on a face of a person and a heart rate of the person is examined.
FIG. 8 is an entire configuration view of a biological information extracting system according to a second embodiment.
FIG. 9 is a function block diagram of the biological information extracting system according to the second embodiment.
FIG. 10 is an entire configuration view of a biological information extracting system according to a third embodiment.
FIG. 11 is a function block diagram of the biological information extracting system according to the third embodiment.
FIG. 12 is a schematic view of biological information extracting system 1 according to a fourth embodiment.
FIG. 13 is a view illustrating a modification example of a camera of a biological information extracting device.

### DESCRIPTION OF EMBODIMENTS

Hereinafter, embodiments of the present disclosure will be described with reference to the drawings. However, detailed explanation may be omitted more than necessary. For example, there are cases where detailed explanation of well-known matters and redundant explanation on substantially the same configuration is omitted. This is to avoid unnecessary redundancy of the following description and to facilitate understanding by those skilled in the art. The accompanying drawings and the following description are provided to enable those skilled in the art to fully understand the present disclosure, and are not intended to limit the subject described in claims.

### (First Embodiment)

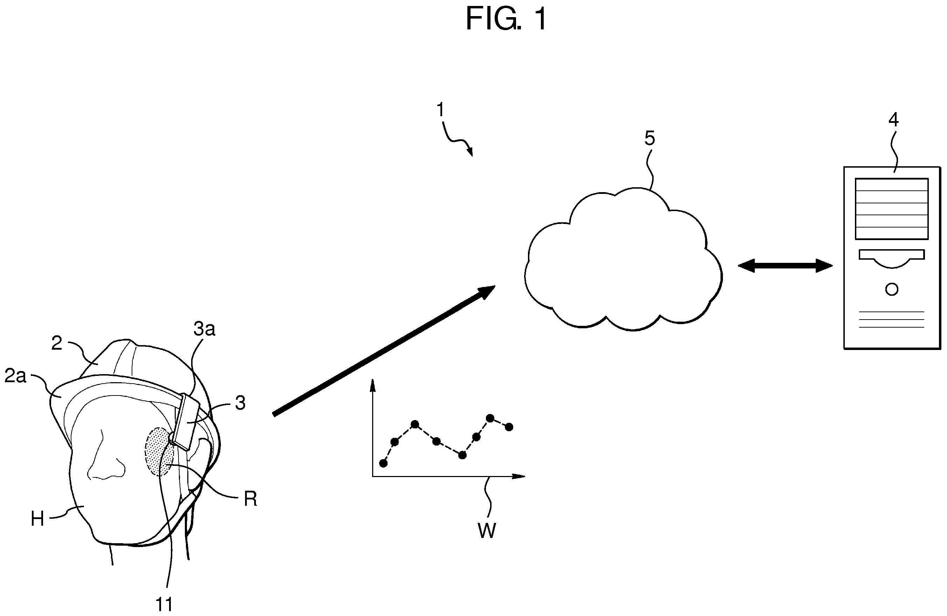

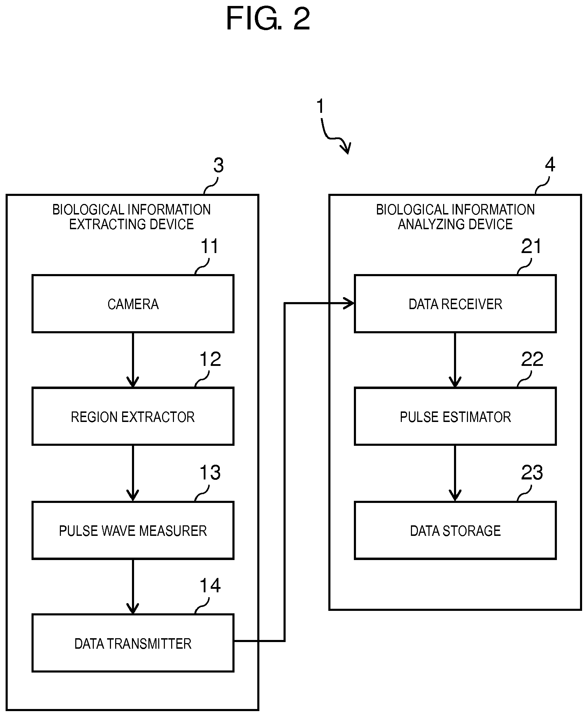

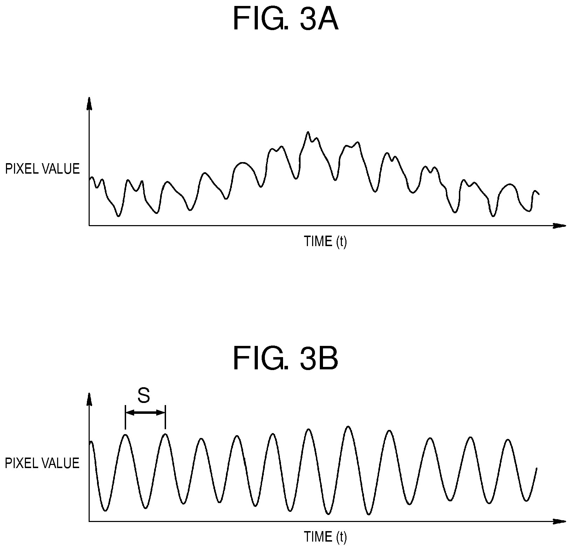

FIGS. 1 and 2 are respectively an entire configuration view and a function block diagram of biological information extracting system 1 according to a first embodiment, and FIG. 3 is an explanatory view of a pulse wave measuring process and a pulse estimating process in biological information extracting device 3 and biological information analyzing device 4 illustrated in FIG. 2.

Biological information extracting system 1 is provided for extracting biological information (for example, a pulse wave or a pulse) from information (captured image) obtained without coming into contact with a human body, and as illustrated in FIG. 1, includes biological information extracting device 3 that is provided in head-mounted member 2 mounted on a head of person (subject) H and extracts pulse wave signal W that is original information of the biological information of person H, and biological information analyzing device 4 that receives pulse wave signal W transmitted from biological information extracting device 3. In biological information extracting system 1, biological information extracting device 3 and biological information analyzing device 4 are communicably connected to each other via wireless communication network 5 such as a wireless Local Area Network (LAN) or a Bluetooth (registered trademark). However, the configuration is not limited thereto and biological information extracting device 3 and biological information analyzing device 4 may be wire-connected by a known communication cable.

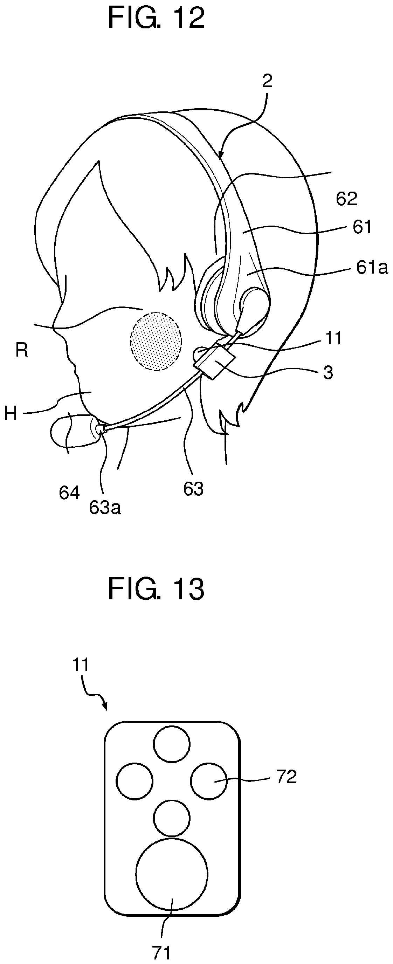

In the embodiment, person H is a worker at a construction site, a building site, or the like and head-mounted member 2 is a helmet. Hereinafter, head-mounted member 2 is also referred to as "helmet". In the example of FIG. 1, helmet 2 has flange 2a formed over an entire circumference at a lower end thereof. Person H is not limited to the worker at the construction site, the building site, or the like and may be, for example, operators of various heavy machines such as a crane and a shovel carrier, drivers of various vehicles such as a truck and a general car, a phone operator of a call center (see FIG. 12), or the like. In addition, head-mounted member 2 is not limited to the helmet and may be a headset (see FIG. 12), a head gear, a cap, or the like.

As illustrated in FIG. 2, biological information extracting device 3 includes camera 11 that captures temporally successive images including at least a part of a face of person H as an object, region extractor 12 that extracts a skin color region from the image captured by camera 11, pulse wave measurer 13 that measures pulse wave signal W of person H based on the skin color region extracted by region extractor 12, and data transmitter 14 that transmits pulse wave signal W measured by pulse wave measurer 13 via wireless communication network 5.

Biological information extracting device 3 has a thin and substantially rectangular housing, and accommodates therein hardware for executing a function of each unit which is described later. A lens of camera 11 is exposed on a side surface of the housing. Upper end portion 3a of biological information extracting device 3 is fixedly attached to a portion on the side of the face of person H at flange 2a of helmet 2 by using a known attachment mechanism (not illustrated). Therefore, camera 11 is disposed toward the side surface of the face of person H. In the example of FIG. 1, camera 11 is disposed to face the side surface of the face of person H. The attachment mechanism described above includes, for example, an attachment mechanism having an engagement portion of a U-shaped cross section, which can be fixed by inserting flange 2a. As long as camera 11 is disposed to face the side surface of the face of person H, an attaching method of biological information extracting device 3 to helmet 2 is not particularly limited. For example, a pair of engagement mechanisms capable of engaging with each other, in which one is disposed at helmet 2 and the other is disposed in biological information extracting device 3, may be used. Alternatively, helmet 2 and biological information extracting device 3 may be integrally configured.

Camera 11 is a camera having a known configuration and includes an image pickup element for converting light of a formed image into an electric signal by forming an image of light from the object (person H) obtained through the lens in an image sensor (CCD, CMOS, or the like) which is not illustrated. As described above, since camera 11 is disposed to face the side surface of the face of person H, camera 11 captures an image of the side surface of the face of person H. In addition, image capturing region R of camera 11 is set not to an entire side of the side surface of the face of person H but to a part of the side surface of the face. Therefore, image data capable of identifying the face of person H is not created.

In the embodiment, the placing position of camera 11 is set so as to capture an image of a cheek of the face of person H. That is, image capturing region R of camera 11 becomes the cheek of the face. As will be described in detail later, in a biological information acquisition technique used in the present disclosure, pulse wave signal W as the time series data is extracted based on a captured image of the skin of person H captured by camera 11. Pulse wave signal W is a signal extracted based on a change in a light absorption amount (specifically, a hemoglobin concentration in the blood) caused by a change in a volume of the blood vessel due to contraction of the heart. Therefore, it is preferable that image capturing region R is a thin portion of the skin so that capturing of the image of the skin of person H is easy and the change in the light absorption amount can be accurately extracted. The cheek of the face is flat, an area thereof is wide, the skin is thin, so that the cheek of the face is optimal as image capturing region R for extracting pulse wave signal W. Pulse wave signal W of person H can be accurately extracted by setting the cheek of the face as image capturing region R.

In a case where region extractor 12 obtains a representative value representing the color (or luminance) of a pixel block for each pixel block of a predetermined size in a captured image (frame image), and the representative value coincides with a predetermined skin color value, the pixel block or a region within a predetermined range from the pixel block is extracted as the skin color region. The skin color value is automatically set based on a representative value of a skin color candidate region measured by using a known image recognition technique from the captured image. Moreover, the skin color value varies depending on each individual and a light condition when an image is captured, so that the skin color value may be set when person H wears helmet 2 and at each predetermined time interval.

Pulse wave measurer 13 calculates, for example, pixel values (0-255 gradations) of each component of RGB regarding each pixel configuring the skin color region extracted in the temporarily continuous captured image, and generates time series data of a representative value (here, an average value of respective pixels) as pulse wave signal W. In this case, it is possible to generate pulse wave signal W that is the time series data based on a pixel value of only a green component (G) of which variation is particularly large due to pulsation. Pulse wave signal W is wave form data (see FIG. 3A).

Data transmitter 14 can transmit the biomaterial-related data (in the embodiment, pulse wave signal W generated by pulse wave measurer 13) regarding a biomaterial of person H extracted based on the skin color region by using a known wireless communication technique such as a wireless LAN or a Bluetooth (registered trademark) to biological information analyzing device 4. Moreover, the wireless communication technique used in data transmitter 15 is not particularly limited and various known wireless communication techniques can be used.

As illustrated in FIG. 2, biological information analyzing device 4 includes data receiver 21 that receives the biomaterial-related data (in the embodiment, pulse wave signal W) transmitted from biological information extracting device 3, pulse estimator 22 that estimates the pulse of person H based on pulse wave signal W received by data receiver 21, and data storage unit 23 that stores the pulse estimated by pulse estimator 22 as pulse data M.

Similar to data transmitter 15 of biological information extracting device 3, data receiver 21 can receive the biomaterial-related data transmitted from data transmitter 15 by using a known wireless communication technique such as a wireless LAN or a Bluetooth (registered trademark). Moreover, the wireless communication technique used by data receiver 21 is not particularly limited as long as the biomaterial-related data transmitted from data transmitter 15 can be received.

For example, as illustrated in FIG. 3A, pulse wave signal W that is the time series data of the pixel value (average value) may be a minute variation (for example, variation less than one gradation of the pixel value) based on a change in hemoglobin concentration in the blood. Therefore, pulse estimator 22 performs a known filtering process (for example, a process by a band filter in which a predetermined passband is set, or the like) with respect to pulse wave signal W, so that as illustrated in FIG. 3B, it is possible to extract pulse wave signal W from which a noise component is removed. Thereafter, pulse estimator 22 can calculate interval S (see FIG. 3B) between adjacent peaks in pulse wave signal W from which the noise component is removed, that is, an interval (pulse wave interval) S of a cycle of one beat of the pulse wave, and estimate the pulse (pulse rate) based on pulse wave interval S. Since pulse wave interval S is caused by the pulsation of the heart, pulse wave interval S can be regarded as corresponding to a heart beat interval (RRI) in an electrocardiograph. Therefore, it is possible to estimate the heart rate based on pulse wave interval S.

An estimated result (heart rate) in pulse estimator 22 is stored in data storage unit 23 as pulse data. In addition, various information (for example, a wave form of the pulse wave or the like) including the estimated result by pulse estimator 22 is output and displayed on a display device (known display device) which is not illustrated.

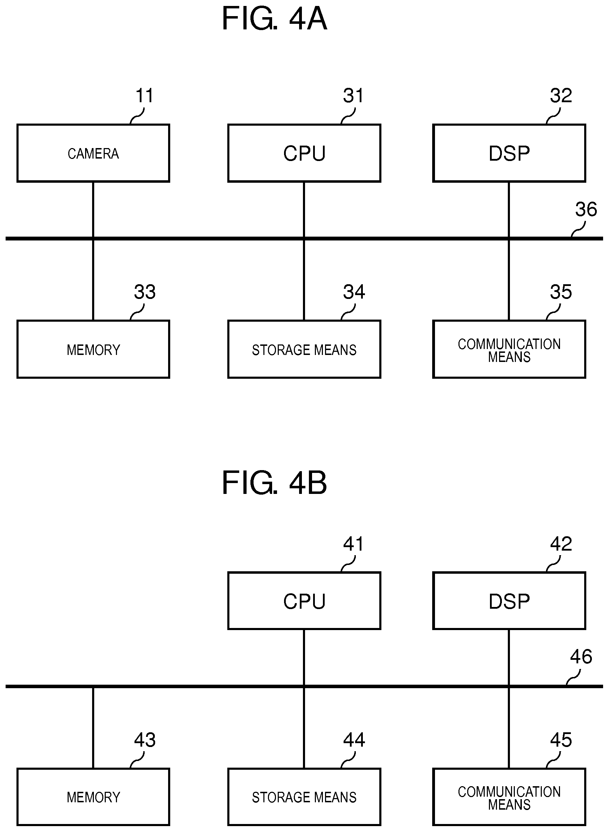

FIG. 4A is a block diagram illustrating a hardware configuration for realizing biological information extracting device 3 according to the first embodiment. Biological information extracting device 3 has a hardware configuration including camera 11, Central Processing Unit (CPU) 31 that collectively executes various information processes (for example, a region extraction process and a pulse wave measurement process), control of peripheral devices, or the like based on a predetermined control program, Digital Signal Processor (DSP) 32, memory 33 such as a Random Access Memory (RAM) that functions as a wok area of CPU 31 and DSP 32, or a Read Only Memory (ROM) that stores a control program executed by CPU 31 and DSP 32 and data, storage means 34 such as a Hard Disk Drive (HDD) or a memory card for accumulating a processing result by biological information extracting device 3, communication means 35 such as a wireless LAN or a Bluetooth (registered trademark) for performing wireless communication with biological information analyzing device 4, and bus 36 that connects these units. At least a part of the function of each unit of biological information extracting device 3 illustrated in FIG. 2 can be realized by executing a predetermined control program by CPU 31 and DSP 32. At least a part of the function of biological information extracting device 3 may be replaced by other known hardware.

FIG. 4B is a block diagram illustrating a hardware configuration for realizing biological information analyzing device 4 according to the first embodiment. Biological information analyzing device 4 has Central Processing Unit (CPU) 41 that collectively executes various information processes (for example, a region extraction process and a pulse wave measurement process), control of peripheral devices, or the like based on a predetermined control program, Digital Signal Processor (DSP) 42, memory 43 such as a Random Access Memory (RAM) that functions as a wok area of CPU 41 and DSP 42, or a Read Only Memory (ROM) that stores a control program executed by CPU 41 and DSP 42 and data, storage means 44 such as a Hard Disk Drive (HDD) or a memory card for accumulating a processing result by biological information analyzing device 4, communication means 45 such as a wireless LAN or a Bluetooth (registered trademark) for performing wireless communication with biological information extracting device 3, and bus 46 that connects these units. At least a part of the function of each unit of biological information analyzing device 4 illustrated in FIG. 2 can be realized by executing a predetermined control program by CPU 41 and DSP 42. At least a part of the function of biological information analyzing device 4 may be replaced by other known hardware.

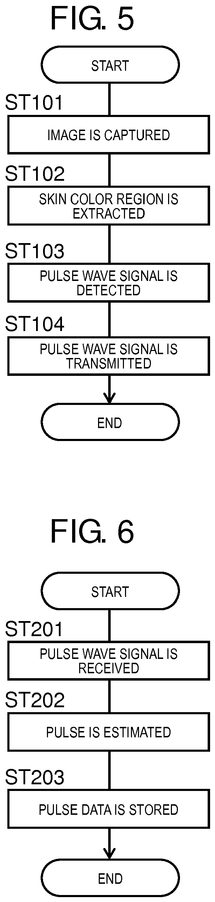

Next, flows of processes by biological information extracting device 3 and biological information analyzing device 4 according to the first embodiment are respectively described with reference to flowcharts of FIGS. 5 and 6.

FIG. 5 is a flowchart illustrating a flow of a process by biological information extracting device 3 according to the first embodiment. First, camera 11 captures the captured image which is temporarily continuous captured image including at least a part (in the embodiment, the cheek of the face) of the face of person H as the object (ST101). Subsequently, region extractor 12 extracts the skin color region in the captured image (frame image) captured by camera 11 (ST102). Next, pulse wave measurer 13 generates the time series data as pulse wave signal W based on each pixel value configuring the skin color region extracted by region extractor 12. In ST104, data transmitter 14 transmits pulse wave signal W generated by pulse wave measurer 13 to biological information analyzing device 4 as the biomaterial-related data (ST104). In biological information extracting device 3, the above-described steps ST101 to ST104 are repeatedly executed in order.

FIG. 6 is a flowchart illustrating a flow of a process by biological information analyzing device 4 according to the first embodiment. First, data receiver 21 receives pulse wave signal W from data transmitter 14 of biological information extracting device 3 (ST201). Subsequently, pulse estimator 22 measures the pulse wave by executing a known filter process with respect to pulse wave signal W, calculates interval S (see FIG. 3B) between the peaks adjacent to each other in the pulse wave, that is, interval (pulse wave interval) S of a cycle of one beat of the pulse wave, and estimates the pulse (pulse rate) based on pulse wave interval S (ST202). Data storage unit 23 stores the pulse estimated by pulse estimator 22 as the pulse data (ST203). Moreover, in biological information analyzing device 4, the above-described steps ST201 to ST203 are repeatedly executed in order.

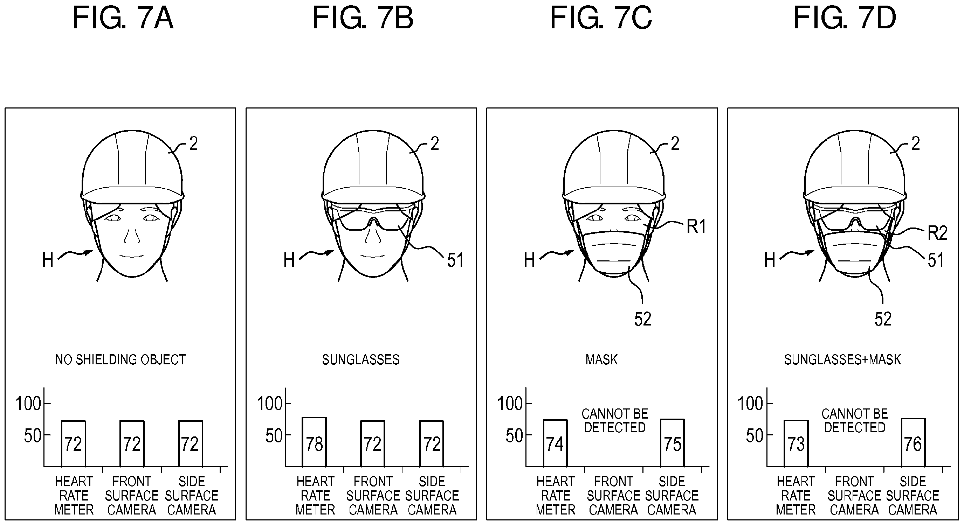

FIGS. 7A to 7D are views illustrating a result of an experiment in which a relationship between a shielding object (sunglasses 51 or mask 52) worn on the face of person H and the heart rate of person H is examined. FIG. 7A illustrates a case where person H does not wear the shielding object, FIG. 7B illustrates a case where person H wears sunglasses 51 on the face, FIG. 7C illustrates a case where person H wears mask 52 on the face, and FIG. 7D illustrates a case where person H wears sunglasses 51 and mask 52 on the face. In addition, in FIGS. 7A to 7D, a "heart rate meter" indicates a heart rate measured by the heart rate meter, a "front surface camera" indicates a heart rate measured based on the captured image obtained by capturing the front surface of the face of person H, and a "side surface camera" indicates a heart rate measured based on the captured image obtained by capturing the side surface of the face of person H, respectively.

As illustrated in FIGS. 7A to 7D, the heart rate (side surface camera) measured based on the captured image obtained by capturing the side surface of the face of person H was substantially equal to the heart rate (heart rate meter) measured by the heart rate meter even in a case where person H wears the shielding object on the face (FIGS. 7B, 7C, and 7D) and even in a case where person H does not wear the shielding object on the face (FIG. 7A). On the other hand, the heart rate (front surface camera) measured based on the captured image obtained by capturing the front surface of the face of person H could not be measured in a case where person H wears mask 52 on the face (FIG. 7C) and in a case where person H wears sunglasses 51 and mask 52 on the face (FIG. 7D). From the above fact, it was found that in a case where person H wears mask 52, the heart rate of person H cannot be measured from the captured image obtained by capturing the front surface of the face of person H, but can be measured from the captured image obtained by capturing the side surface of the face of person H. Therefore, it was found that it is better to capture the side surface of the face of person H than the front surface thereof in consideration of a case where person H wears the shielding object such as mask 52 on the face.

As described above, according to biological information extracting system 1 according to the first embodiment, a configuration, in which the side surface of the face of person H is captured by camera 11 disposed toward the side surface of the face of person H, is provided, so that even in a case where person H wears the shielding object such as the sunglasses or the mask on the face, it is possible to extract the biological information of person H. In addition, a configuration, in which an image of a part (in the embodiment, the cheek of the face) of the side surface of the face of person H is captured by camera 11, is provided, so that image data capable of identifying the face of person H is not created. Therefore, even in a case where troubles such as erroneous transmission of the image data and information leakage occur, it is possible to protect the privacy of person H. Furthermore, a configuration, in which pulse wave signal W is generated in biological information extracting device 3 and is transmitted to biological information analyzing device 4, is provided, so that it is possible to greatly reduce the transmission data amount without lowering the extraction accuracy of the biological information compared to a case where the image data is transmitted.

In the embodiment, image capturing region R of camera 11 is the cheek of the face, but image capturing region R may be a portion other than the cheek of the face as long as image capturing region R is a part of the side surface of the face of person H. For example, region R1 above mask 52 on the face of person H as illustrated in FIG. 7C, region R2 between mask 52 and sunglasses 51 on the face of person H as illustrated in FIG. 7D, a temple portion of person H, a region on the front side of the ear of person H, or the like can be taken as image capturing region R.

In addition, in the first embodiment, in order to reduce a processing amount in biological information extracting device 3, pulse wave signal W before executing the filtering process is transmitted from biological information extracting device 3 to biological information analyzing device 4. However, the pulse wave signal (that is, the pulse wave signal from which noise component is removed) after executing the filtering process may be transmitted. Also in this case, it is possible to greatly reduce the transmission data amount compared to a case where the image data is transmitted. Moreover, in this case, the filtering process of pulse wave signal W is executed in biological information extracting device 3.

### (Second Embodiment)

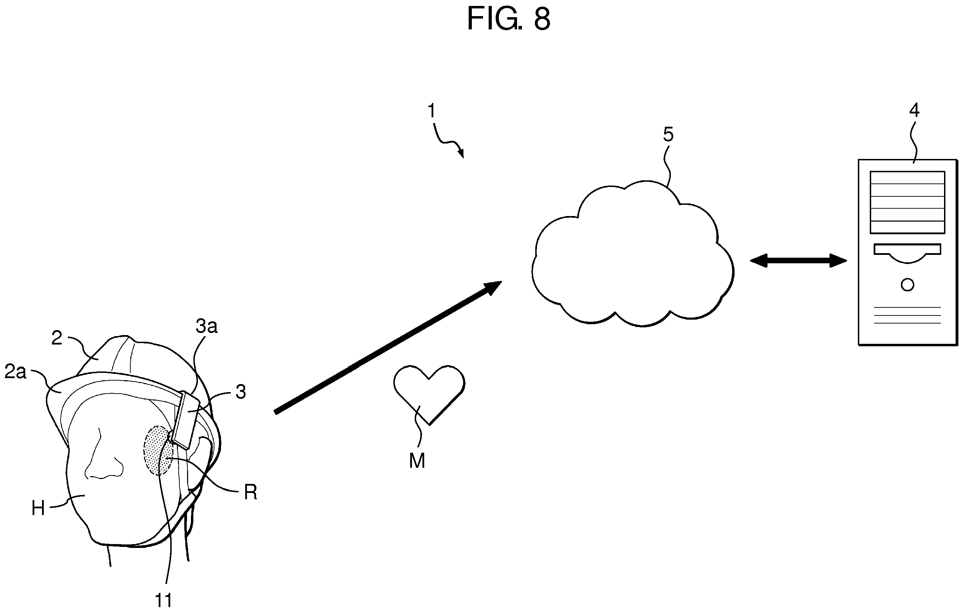

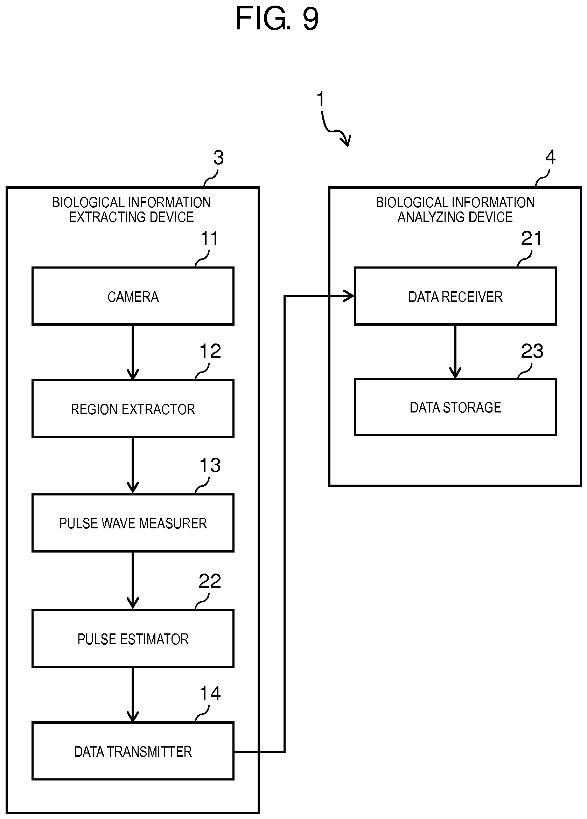

FIGS. 8 and 9 are respectively an entire configuration view and a function block diagram of biological information extracting system 1 according to a second embodiment. In FIGS. 8 and 9, the same reference numerals are given to the same configuration elements as those of the first embodiment which is described above. In addition, in the second embodiment, matters not specifically mentioned are the same as those in the case of the first embodiment which is described above.

As illustrated in FIG. 8, biological information extracting system 1 according to the second embodiment is different from that of the case of the first embodiment in that pulse data M as biomaterial-related data is transmitted from biological information extracting device 3 to biological information analyzing device 4. In addition, as illustrated in FIG. 9, pulse estimator 22 being provided in biological information extracting device 3 instead of biological information analyzing device 4 is different from that of the case of the first embodiment. In biological information extracting system 1 according to the second embodiment, the estimation of the pulse (pulse rate) is performed in biological information extracting device 3 and pulse data M is transmitted from biological information extracting device 3 to biological information analyzing device 4. Biological information analyzing device 4 stores pulse data M received from biological information extracting device 3 in data storage unit 23.

According to biological information extracting system 1 according to the second embodiment, pulse data M is generated in biological information extracting device 3 and pulse data M is transmitted to biological information analyzing device 4, so that it is possible to greatly reduce a transmission data amount without lowering the extraction accuracy of the biological information compared to a case where the image data is transmitted. In addition, since it is possible to transmit pulse data M which is the biological information from biological information extracting device 3, a degree of freedom of the configuration of biological information extracting system 1 is enhanced. Therefore, it is possible to construct biological information extracting system 1 having various configurations. In addition, a configuration, in which an image of a part (in the embodiment, the cheek of the face) of the side surface of the face of person H is captured by camera 11 disposed toward the side surface of the face of person H, is provided, so that similar to the case of the first embodiment which is described above, it is possible to protect the privacy of person H and extract biological information of person H even in a case where person H wears a shielding object such as sunglasses or a mask on the face.

### (Third Embodiment)

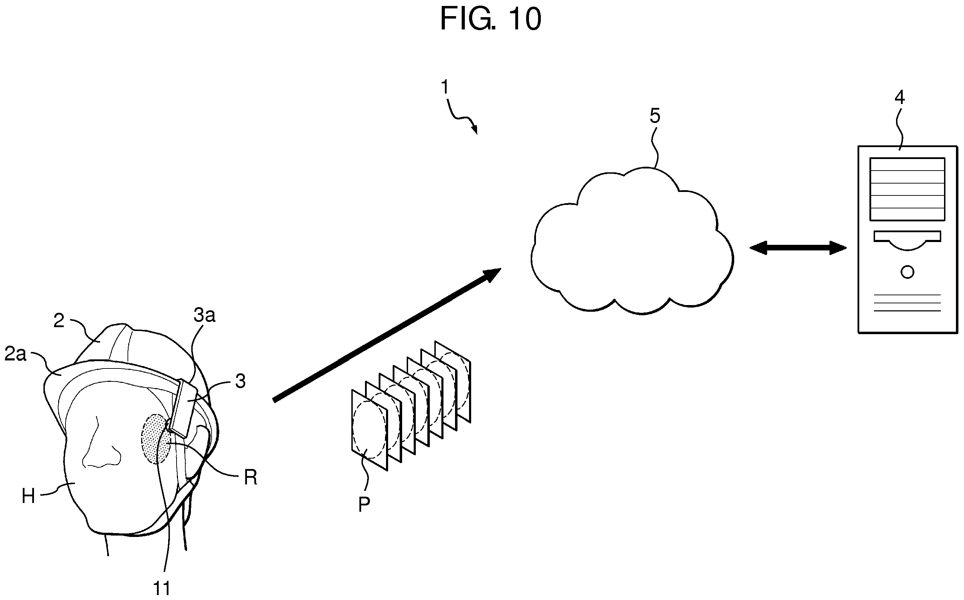

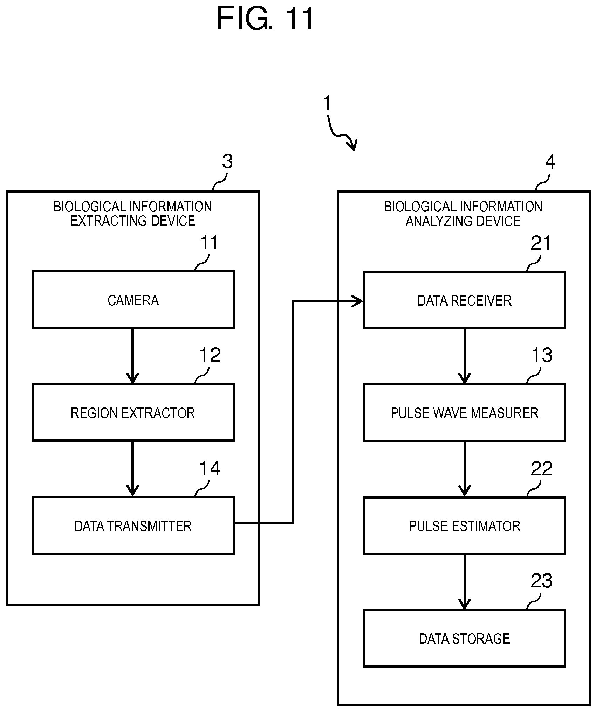

FIGS. 10 and 11 are respectively an entire configuration view and a function block diagram of biological information extracting system 1 according to a third embodiment of the present disclosure. In FIGS. 10 and 11, the same reference numerals are given to the same configuration elements as those of the first embodiment which is described above. In addition, in the third embodiment, matters not specifically mentioned are the same as those in the case of the first embodiment which is described above.

As illustrated in FIG. 10, biological information extracting system 1 according to the third embodiment is different from that of the case of the first embodiment in that image data P as biomaterial-related data is transmitted from biological information extracting device 3 to biological information analyzing device 4. In addition, as illustrated in FIG. 11, pulse wave measurer 13 being provided in biological information analyzing device 4 instead of biological information extracting device 3 is different from that of the case of the first embodiment. In biological information extracting system 1 according to the third embodiment, image data P is transmitted from biological information extracting device 3 to biological information analyzing device 4, and measurement of the pulse wave signal and estimation of the pulse (pulse rate) based on the pulse wave signal are performed in biological information analyzing device 4.

According to biological information extracting system 1 according to the third embodiment, image data P that is the time series image data of the skin color region is generated in biological information extracting device 3 and pulse data P is transmitted to biological information analyzing device 4, so that it is possible to greatly reduce a transmission data amount without lowering the extraction accuracy of the biological information compared to a case where the whole face image is transmitted. In addition, a configuration, in which an image of a part (in the embodiment, the cheek of the face) of the side surface of the face of person H is captured by camera 11 disposed toward the side surface of the face of person H, is provided, so that similar to the case of the first embodiment which is described above, it is possible to protect the privacy of person H and extract biological information of person H even in a case where person H wears a shielding object such as sunglasses or a mask on the face.

### (Fourth Embodiment)

FIG. 12 is a schematic view of biological information extracting system 1 according to a fourth embodiment. In FIG. 12, the same reference numerals are given to the same configuration elements as those of the first embodiment which is described above. In addition, in the first embodiment, matters not specifically mentioned are the same as those in the case of the first embodiment which is described above. In FIG. 12, biological information analyzing device 4 is not illustrated.

Biological information extracting system 1 according to the fourth embodiment is different from that of the case of the first embodiment in that headset 2 is used instead of helmet 2 as the head-mounted member. Headset 2 includes band portion 61 mounted on the head of person H, headphone 62 that is disposed at lower end 61a of band portion 61, arm 63 that extends from lower end 61a of band portion 61, and microphone 64 that is disposed at tip 63a of arm 63. Biological information extracting device 3 is fixedly attached to a portion positioned toward the cheek of the face of person H in arm 63 by using a known attachment mechanism (not illustrated) such as a clip. The placing position of camera 11 is set so as to capture an image of the cheek of the face of person H. That is, image capturing region R of camera 11 becomes the cheek of the face.

Biological information extracting system 1 according to the fourth embodiment can be applied to a telephone operator of a call center or the like. According to biological information extracting system 1 according to the fourth embodiment, a configuration, in which an image of a part (in the embodiment, the cheek of the face) of the side surface of the face of person H is captured by camera 11 disposed toward the side surface of the face of person H, is provided, so that similar to the case of the first embodiment which is described above, it is possible to protect the privacy of person H and extract biological information of person H even in a case where person H wears a shielding object such as sunglasses or a mask on the face.

FIG. 13 is a view illustrating a modification example of camera 11 of biological information extracting device 3. As illustrated in FIG. 13, camera 11 includes lens 71 and LED lighting 72. Lens 71 includes a visible light cut filter (not illustrated) that transmits infrared light and blocks visible light, and an infrared light cut filter (not illustrated) that transmits visible light and blocks infrared light, and is configured to switch the filters in accordance with brightness detected by an illuminance sensor (not illustrated). Therefore, it is possible to capture an image with visible light as a light source in the daytime and to capture an image with infrared light as a light source in nighttime. Therefore, it is possible to capture person H more clearly in the daytime and in nighttime. In addition, LED lighting 72 is also driven in accordance with the brightness detected by the illuminance sensor (not illustrated). Therefore, even in a case where a light amount necessary for capturing the image of person H is insufficient, it is possible to capture an image of person H more clearly by driving LED lighting 72 and securing the light amount necessary for capturing an image.

Although the present disclosure is described based on specific embodiments, these embodiments are merely examples, and the present disclosure is not limited by these embodiments. For example, the biological information extracting device and the biological information extracting system according to the present disclosure can be applied not only to a medical application but also to various uses such as face recognition, monitoring, life and death confirmation, and the like. Each configuration element of the biological information extracting device and the biological information extracting system is not necessarily essential, and can be appropriately selected at least as long as the element does not depart from the scope of the present disclosure.

### INDUSTRIAL APPLICABILITY

The biological information extracting device and the biological information extracting system according to the present disclosure are useful as a biological information extracting device, a biological information extracting system, and the like capable of protecting the privacy of the subject, extracting the biological information of the subject even in a case where the subject wears the shielding object such as sunglasses or a mask on the face, and reducing the transmission data amount without lowering the extraction accuracy of the biological information.

### REFERENCE MARKS IN THE DRAWINGS

- 1: BIOLOGICAL INFORMATION EXTRACTING SYSTEM
- 2: HEAD-MOUNTED MEMBER (HELMET, HEADREST)
- 3: BIOLOGICAL INFORMATION EXTRACTING DEVICE
- 4: BIOLOGICAL INFORMATION ANALYZING DEVICE
- 5: COMMUNICATION NETWORK
- 11: CAMERA
- 12: REGION EXTRACTOR
- 14: DATA TRANSMITTER
- 21: DATA RECEIVER
- 22: PULSE ESTIMATOR
- 23: DATA STORAGE UNIT
- 71: LENS
- 72: LED LIGHTING
- H: PERSON (SUBJECT)
- R: IMAGE CAPTURING REGION
- W: PULSE WAVE SIGNAL
- P: IMAGE DATA
- M: PULSE DATA

## Claims

1. A biological information extracting device which is provided at a head-mounted member mounted on a head of a subject and extracts biological information of the subject from information obtained without coming into contact with the subject, the device comprising:
a camera that captures temporally successive images including at least a part of a face of the subject as an object;
a region selector that selects a skin color region from the image captured by the camera; and
a data transmitter that transmits biomaterial-related data relating to a biomaterial of the subject extracted based on the skin color region to a predetermined transmission destination,
wherein the camera is disposed to face a side surface of the face of the subject.

2. The biological information extracting device of Claim 1,
wherein the camera is set to capture a part of the side surface of the face.

3. The biological information extracting device of Claim 2,
wherein a part of the face is a cheek of the face.

4. The biological information extracting device of Claim 1,
wherein the biomaterial-related data is time series image data of the skin color region.

5. The biological information extracting device of Claim 4, further comprising:
a pulse wave measurer that measures a pulse wave signal of the subject based on the time series image data of the skin color region,
wherein the biomaterial-related data is the pulse wave signal.

6. The biological information extracting device of Claim 5, further comprising:
a biological information extractor that extracts biological information of the subject based on the pulse wave signal,
wherein the biomaterial-related data is the biological information.

7. The biological information extracting device of Claim 1,
wherein the head-mounted member includes a helmet or a headrest.

8. The biological information extracting device of Claim 1,
wherein the camera includes a visible light cut filter and an infrared light cut filter on a lens thereof.

9. The biological information extracting device of Claim 1,
wherein the camera includes an LED lighting device.

10. A biological information extracting system comprising:
the biological information extracting device of Claim 1; and
a biological information analyzing device that receives biomaterial-related data transmitted from the biological information extracting device.
